(19) Europäisches Patentamt
European Patent Office
Office européen des brevets

(11) Publication number: **0 147 223**
**A2**

# (12) EUROPEAN PATENT APPLICATION

(21) Application number: **84309064.8**

(22) Date of filing: **21.12.84**

(51) Int. Cl.⁴: **A 01 N 55/04**
A 01 N 47/44, A 01 N 43/78
A 01 N 43/76, A 01 N 43/32
A 01 N 37/40, A 01 N 35/02
A 01 N 31/08, A 61 L 2/18

(30) Priority: **23.12.83 GB 8334423**

(43) Date of publication of application:
**03.07.85 Bulletin 85/27**

(84) Designated Contracting States:
**AT BE CH DE FR IT LI LU NL SE**

(71) Applicant: **STERWIN AG.**
**Zeughausgasse 9**
**CH-6300 Zug(CH)**

(72) Inventor: **Brocklehurst, Peter**
**87 Cowley Lane**
**Chapeltown Sheffield S30 4SX(GB)**

(72) Inventor: **Scaife, Rodney**
**4 Habershon Drive**
**Chapeltown Sheffield S30 4ZT(GB)**

(74) Representative: **Green, Alan James et al,**
**Sanderson & Co. 97 High Street**
**Colchester Essex C01 1TH(GB)**

## (54) A compostition for use as a preservative or disinfectant.

(57) The invention relates to a composition for use as a preservative or disinfectant.

The composition of the invention comprises one or more organic biocides selected from phenols, aldehydes, heterocyclic compounds, quaternary ammonium compounds and cationic compounds, and at least one hydroperoxide of the formula:

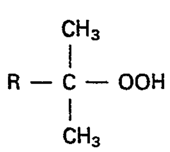

wherein R is an aliphatic or aromatic hydrocarbon group.

In addition, in a modification the invention provides a wood preservative composition comprising said hydroperoxide and a biocide such as tri-butyl tin oxide, together with a non-aqueous carrier or solvent.

EP 0 147 223 A2

# A COMPOSITION FOR USE AS A PRESERVATIVE OR DISINFECTANT

The present invention relates to a composition for use as a preservative or disinfectant, in particular to a composition for use in preserving cutting oils and coolants, or as a disinfectant and, in a modification, for use in preserving wood.

Cutting oils and coolants are used in light engineering to keep tools cool and lubricated during machining, for example, in the sharpening of a chisel using a grinding wheel. Cutting oils generally are oil in water emulsions which can be used as growing media by micro-organisms. In these media micro-organisms can grow to a contamination level of greater than $10^7$ organisms per millilitre, and can cause the oil in water emulsion to break because they use the emulsifier which stabilizes the emulsion as a nutrient. This prevents the liquid from functioning effectively, and moreover, the breakdown products may be acidic and therefore corrosive. As a result tools can be ruined by machining at an incorrect temperature, and corroded by acidic breakdown products. Furthermore, the contaminating organisms can be pathogenic and as such can be a health hazard, especially since cutting oils are sprayed during use.

The breakdown of cutting oils and like coolants has been a major cause for concern over the past ten years and various biocides have been produced to be used specifically in cutting oils. These biocides generally are either phenolic, or are formaldehyde release agents or isothiazolinones, although other less well known types have been developed.

We have now found surprisingly that the activity of well known and commonly used biocides can be enhanced and a synergistic effect obtained by utilizing them in conjunction with specific hydroperoxide compounds. In particular, we have found that certain hydroperoxides not only increase the activity of known biocides as used in cutting oils and coolants but also impart inhibition properties to biocides which normally do not exhibit biostasis. In addition, we have found that compositions for use both as domestic and industrial disinfectants may be formulated utilising a combination of a biocide

and selected hydroperoxides.

Accordingly, the present invention provides a composition for use as a preservative or disinfectant, which composition comprises one or more organic biocides selected from phenols, aldehydes, heterocyclic compounds, quaternary ammonium compounds and cationic compounds, and at least one hydroperoxide of the formula:

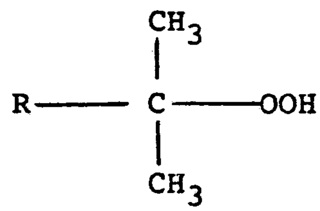

I

wherein R is an aliphatic or aromatic hydrocarbon group.

The composition of the invention may be formulated as a simple mixture comprising its two essential ingredients, or together with a suitable carrier or solvent. In particular, the composition may be formulated as an aqueous emulsion or dispersion, preferably one in which a surface active agent is present in an amount of at least about 10% by weight of the composition.

When the composition of the invention is to be used as a preservative in cutting oils or coolants, the organic biocide is preferably one of those known as suitable for use in cutting oils or coolants. Furthermore, when the composition is to be used as a disinfectant the biocide must be one which is suitable in the chosen industrial or domestic context. However, those criteria having been met the organic biocide may be any biocide selected from the classes defined above, that is to say selected from:

(1) Phenols, including phenol itself, naphthols and indanols, all of which may be substituted or unsubstituted, and particularly including halogenated phenols.

(2) Aldehydes, for example, glyoxal or formaldehyde, including formaldehyde release agents.

(3) Heterocyclic compounds, generally ones containing no more than one sulphur atom and/or containing no aromatic substituents e.g. substituted by alkyl, alkoxy or halo only, such as a dioxane, an

isothiazolinone or an oxazolidine, for example, a dimethoxane or a halo- and/or alkyl-substituted isothiazolinone.

(4) Quaternary ammonium compounds, in particular halides such as bromides or chlorides, or methosulphates.

(5) Cationic compounds, in particular guanides or biguanides such as chlorhexidine, especially organic salts thereof such as chlorhexidine acetate and chlorhexidine gluconate, and related compounds.

Preferably, however, the biocide is an aldehyde, a dioxane, an isothiazolinone, and/or a phenol.

More preferably, the biocide is a phenol of the formula:

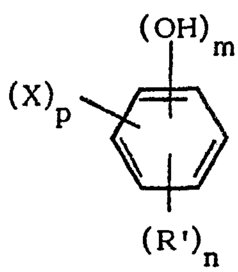

II

wherein m and n are the same or different and are each 1, 2 or 3, p is 0, 1, 2 or 3, R' is hydrogen, alkyl, alkoxy, hydroxyalkyl, aryl, alkaryl or aralkyl, and X is halogen. In such a compound any alkyl, alkoxy or hydroxyalkyl groups are preferably ones containing from 1 to 7 carbon atoms.

In the case where the organic biocide is a phenol of formula II, in one embodiment R' is preferably alkyl, more preferably $C_1$ to $C_7$ alkyl, or aryl, p is 0 and m is 1. Alternatively, in another embodiment, it is preferred that for the phenol of formula II R' is hydrogen or alkyl, m and n are 1 and X is bromo, chloro or iodo.

Thus, in one embodiment preferred compositions of the invention may contain one or more alkyl phenols such as one or more of a heptyl phenol, a propyl phenol, e.g. that sold commercially as a mixture of propylated phenols, o-, m- or p-cresol, or a xylenol; or an aryl phenol such as o-phenylphenol. Furthermore, in another embodiment, preferred compositions of the invention may contain one or more of 4-chlorophenol, 2,4-dichlorophenol, 2,4,6-trichlorophenol, or 4-chloro-o-cresol.

Additionally or alternatively, other phenolic compounds may be used. For example, the compositions of the invention may comprise o-benzyl-p-chlorophenol as biocide, or one or more alkyl p-hydroxybenzoates such as methyl, ethyl, propyl or butyl p-hydroxybenzoate or mixtures thereof.

The compositions of the invention may be prepared in any convenient form, but preferably they are in liquid form. In particular, the compositions may comprise water as a liquid carrier for the biocide and hydroperoxide, together with a surface active agent, preferably present in an amount of at least about 10% by weight, and typically sufficient to establish an emulsion or dispersion of the chosen biocide and hydroperoxide in an aqueous medium. More preferably, the surface active agent is present in an amount of from about 15% to about 35% by weight, most preferably from about 20% to about 30% by weight.

In the compositions of the invention, the surface active agent may be a detergent or a soap, and may be anionic, cationic or non-ionic. The surface active agent, however, must be compatible with the intended use of the composition and thus, for example, with the cutting oil (which itself will usually contain a surface active agent), or the coolant (which typically itself will comprise water and a detergent), with which the composition may be employed. Preferably, the surface active agent is an alkali metal salt of an alkyl sulphate, e.g. a sodium salt of a secondary alkyl sulphate such as that sold under the trade name Teepol, a sodium salt of a primary alkyl sulphate e.g. sodium lauryl sulphate, a sulphonated castor oil, or a mixture thereof. The surface active agent may be specifically chosen so that it is non-foaming, depending on the requirements to be met by the composition, and emulsifying systems such as those comprising sulphonated castor oil may be used as well as more conventional surface active agents.

In the composition of the invention, the hydroperoxide of formula I is preferably tertiary butyl hydroperoxide (R=methyl), tertiary amyl hydroperoxide (R=ethyl) and/or cumene hydroperoxide (R=phenyl). Most

preferably, however, and except as mentioned below, the hydroperoxide is tertiary butyl hydroperoxide.

The composition of the invention generally may comprise any compatible mixture of the biocide and the hydroperoxide. Typically, however, a composition in accordance with the invention formulated with a carrier or solvent, and which is to be used as a preservative, will include up to about 15% by weight of organic biocide, and preferably no more than about 5% by weight of hydroperoxide of formula I. On the other hand disinfectant compositions formulated with a carrier or solvent may contain up to about 40% by weight of biocide.

As a lower limit it may be said that the composition will generally include at least about 0.1% by weight of organic biocide, a preferred range being from about 0.1% to about 10% e.g. about 5%, by weight of organic biocide, especially when used as a preservative or domestic disinfectant. In addition, the composition will more preferably include not more than about 1% by weight of hydroperoxide of formula I, again especially when used as a preservative or domestic disinfectant.

Generally, the weight ratio of organic biocide to hydroperoxide of formula I may range from about 100:1 to about 1:25, typically from about 25:1 to about 1:25, especially in the context of a preservative, with a preferred range of weight ratios being from about 15:1 to about 1:15. In the compositions of the invention, the ratio of biocide to hydroperoxide will depend to an extent mainly on the solubility of the biocide in the chosen solvent e.g. water, and on the type and spectrum of activity required. Thus, where fungicidal and/or fungistatic activity is generally preferred over bactericidal and/or bacteriostatic activity the hydroperoxide may be employed in excess over the biocide, whereas the biocide may be employed in excess over the hydroperoxide when bactericidal etc. activity is preferred to fungicidal etc. activity. In either event, however, the hydroperoxide will boost the activity of the biocide vs. fungi.

Generally speaking, the biocide and hydroperoxide will not be employed in equal amounts. Accordingly, in the above embodiment where bactericidal etc. activity is required the weight ratio of biocide to hydroperoxide

will usually be at least about 2:1 and preferably will approach about 5:1 or higher, with the converse being true when fungicidal etc. activity is required i.e. the ratio being at least about 1:2, preferably about 1:5 or lower.

The composition of the invention is employed as a preservative by adding a suitable amount of the composition to a cutting oil or coolant either before or after the former has been brought to its own use dilution. Thus, the cutting oil or coolant may be preserved as a concentrate prior to use by employing the disclosed composition, or the composition can be added when the cutting oil or coolant concentrate has been diluted with water in readiness for use.

The composition of the invention may be employed at dilutions in the diluted product to be protected down to about 0.1% by weight, with a preferred range being from about 0.1% to about 0.5% by weight. Generally, however, for control of microorganisms within a bulk concentrated product the composition will be used at dilutions in the range of from about 1% to about 5% by weight.

Alternatively, the compositions of the invention may be employed as disinfectants at use dilutions of about 10% by weight or lower, and preferably again at dilution levels as set out above. When intended for use as disinfectants, especially in an industrial context, the compositions can include stabilizers such as urea, alkali metal metaphosphates, alkali metal polyphosphates, pyridin-2,3-dicarboxylic acid, pyridin-2,6-dicarboxylic acid, citric acid and its alkali metal salts, E.D.T.A. and its alkali metal salts, and nitriloacetic acid and its alkali metal salts (or mixtures thereof), as well as other ingredients known for use in disinfectant compositions. If used, a stabilizer will usually be present in an amount of from about 0.1% to 7% by weight. Also, when intended for use as disinfectants, the compositions may include other conventional ingredients such as alcohols e.g. isopropyl alcohol.

The compositions of the invention are illustrated by the following specific Examples. In all of the Examples which include a surface active agent (Teepol or sulphonated castor oil), the compositions were formulated by blending as follows:

1. Prepare an emulsion of the surface active agent, hydroperoxide (70% aqueous solution) and organic biocide with gentle heating (to less than about 40°C).
2. Add the thus-prepared emulsion to water at from about 30°C to about 40°C until the desired concentration of biocide is obtained.

Also, in all of the Examples, unless indicated otherwise, the percent by weight of hydroperoxide is based on 100% hydroperoxide.

Example 1

A preservative composition was formulated by blending as follows:

| Ingredients | Percent by weight |
|---|---|
| A mixture of xylenols | 5 |
| Tertiary butyl hydroperoxide (TBHP) | 1 |
| Teepol | 20 |
| Water | balance to 100% |

The above composition when added to a 4% cutting oil emulsion (Dromus F) at a concentration of 0.25% passed a 30% challenge test at a minimum level of $10^7$ organisms per ml. On the other hand both TBHP and the biocide (xylenol mixture) exhibited very limited activity in the same test.

Similar results were also obtained with the following mixtures:

Examples 2 to 19

| Example | Ingredients | Percent by weight |
|---|---|---|
| 2 | 2,4-Dichlorophenol | 5 |
| | TBHP | 1 |
| | Teepol | 20 |
| | Water | balance to 100% |

| | | |
|---|---|---|
| 3 | 2,4-Dichlorophenol | 10 |
| | TBHP | 1 |
| | Teepol | 30 |
| | Water | to 100% |
| 4 | 4-Chlorophenol | 5 |
| | TBHP | 1 |
| | Teepol | 20 |
| | Water | balance to 100% |
| 5 | 4-Chlorophenol | 10 |
| | TBHP | 1 |
| | Teepol | 30 |
| | Water | balance to 100% |
| 6 | 2,4,6-Trichlorophenol | 5 |
| | TBHP | 1 |
| | Teepol | 20 |
| | Water | balance to 100% |
| 7 | 2,4,6-Trichlorophenol | 10 |
| | TBHP | 1 |
| | Teepol | 30 |
| | Water | balance to 100% |
| 8 | KATHON RH886 - a mixture of 3 parts of 5-chloro-2-methyl-4-isothiazolin-3-one and 1 part of 2-methyl-4-isothiazolin-3-one containing 15% of active ingredients | 1 |
| | TBHP | 1 |
| | Teepol | 10 |
| | Water | balance to 100% |
| 9 | 4-Chloro-o-cresol | 5 |
| | TBHP | 1 |
| | Teepol | 20 |
| | Water | balance to 100% |

| Example | Ingredients | Percent by weight |
|---|---|---|
| 10 | 4-Chloro-o-cresol | 10 |
| | TBHP | 1 |
| | Teepol | 30 |
| | Water | balance to 100% |
| 11 | o-Phenylphenol | 5 |
| | TBHP | 1 |
| | Teepol | 20 |
| | Water | balance to 100% |
| 12 | o-Phenylphenol | 10 |
| | TBHP | 1 |
| | Teepol | 30 |
| | Water | balance to 100% |
| 13 | A mixture of propylated phenols | 5 |
| | TBHP | 1 |
| | Teepol | 20 |
| | Water | balance to 100% |
| 14 | A mixture of propylated phenols | 10 |
| | TBHP | 1 |
| | Teepol | 30 |
| | Water | balance to 100% |
| 15 | A mixture of xylenols | 10 |
| | TBHP | 1 |
| | Teepol | 30 |
| | Water | balance to 100% |
| 16 | Nipa esters (8+5) - a mixture of ethyl p-hydroxybenzoate and propyl p-hydroxybenzoate in a ratio of 8 to 5 | 10 |
| | TBHP | 1 |
| | Teepol | 20 |
| | Water | balance to 100% |

| Example | Ingredients | Percent by weight |
|---|---|---|
| 17 | Nipa esters (8+5) | 15 |
| | TBHP | 1 |
| | Teepol | 25 |
| | Water | balance to 100% |
| 18 | 6-Acetoxy-2,4-dimethyl-m-dioxane | 5 |
| | TBHP | 1 |
| | Teepol | 20 |
| | Water | balance to 100% |
| 19 | 6-Acetoxy-2,4-dimethyl-m-dioxane | 10 |
| | TBHP | 1 |
| | Teepol | 25 |
| | Water | balance to 100% |

(In all of the above Examples the Teepol may be replaced by sulphonated castor oil in order to give a similar composition.)

| Example | Ingredients | Percent by weight |
|---|---|---|
| 20 | KATHON RH886 | 2.5 |
| | 2-Chlorophenol | 25 |
| | Water | 0.5 |
| | TBHP | 1 |
| | Sulphonated castor oil | 71 |
| 21 | Grotan OX, that is N,N'-methylene-bis(5-methyl-oxazolidine) | 50 |
| | Water | 2 |
| | TBHP | 5 |
| | Sulphonated castor oil | 43 |

(The compositions of Examples 20 and 21 are particularly for use by the cutting oil manufacturer to preserve neat oils. Efficacy studies have shown that upon dilution to the manufacturer's recommended use dilution, the preserved cutting oil will withstand a multiple inoculum challenge of 50% v/v of an inoculum containing $\geqslant 10^6$ organisms per ml using either of the exemplified compositions.)

| Example | Ingredients | Percent by weight |
|---|---|---|
| 22 | KATHON RH886 | 2.5 |
| | 2-Chlorophenol | 25 |
| | Water | 41.5 |
| | TBHP | 1 |
| | Propylene glycol | 30 |
| 23 | Grotan OX | 50 |
| | Water | 45 |
| | TBHP | 5 |

(The compositions of Examples 22 and 23 are particularly for use at concentrations of between 0.1% and 0.2% by the end user of the cutting oil and are to be added directly to the oil at its use dilution. While they are compatible with the neat oil preservatives generally used, the compositions are nevertheless intended to be added to unpreserved cutting oils. Either exemplified composition at a concentration of 0.15% will withstand a 50% inoculum containing $\geqslant 10^6$ organisms per ml.)

In addition to the above preservative compositions, disinfectant compositions were formulated by blending as follows:

Example 24

| Ingredients | Percent by weight |
|---|---|
| Urea | 5.3 |
| Sodium alkyl sulphate | 20 |
| Sodium lauryl sulphate | 6.7 |
| Isopropyl alcohol | 10 |
| Xylenols | 36 |
| Water | 18.4 |
| TBHP | 2 |
| Castor oil soap | 1.6 |

The above composition was tested against the same composition without the TBHP (and including 2% more water) in the Kelsey Sykes capacity test (as modified in 1974) with the following results:

| | Without TBHP | With TBHP |
|---|---|---|
| Clean conditions | 0.6% | 0.6% |
| Dirty conditions | 0.85% | 0.7% |

The above results show a significant improvement in the activity of the composition with TBHP versus the

composition without TBHP under dirty conditions.

Example 25

| Ingredients | Percent by weight |
| --- | --- |
| OBPCP (o-benzyl-p-chlorophenol) | 0.5 |
| TBHP | 1.0 |
| Teepol | 10 |
| Water | balance to 100% |

The above composition was tested against TBHP alone, and against the same composition without the TBHP (and including 1% more water), in the Rideal Walker phenol coefficient test with the following results:

| | With TBHP | Without TBHP | TBHP alone |
| --- | --- | --- | --- |
| RW coefficient | 5.0 | 3.9 | 0 |

Once again a significant and synergistic improvement is shown.

Example 26

| Ingredients | Percent by weight |
| --- | --- |
| Urea | 5.3 |
| Sodium alkyl sulphate | 20 |
| Sodium lauryl sulphate | 6.7 |
| Isopropyl alcohol | 10 |
| Xylenols | 36 |
| Nervanoid 330 (EDTA) | 0.1 |
| Perfume L7496 | 0.6 |
| Water | 17.7 |
| TBHP (70% solution in water) | 2 |
| Castor oil soap | 1.6 |

The above composition provides a clear soluble phenolic disinfectant giving similar results to those shown in Example 24 when tested by the Kelsey Sykes Test (as modified in 1974). The addition of 1.4% TBHP overall to the base formulation increases the activity by some 20% to 25%. Also, the stability of the composition appears to be very good with no significant changes being observed after three months at 37°C in polyethylene or polypropylene containers.

In a modification of the above-described invention, we have also found that a system comprising a

hydroperoxide of the formula I and any suitable biocide may be formulated as a wood preservative. Accordingly, in a modification the composition of the invention may be formulated as a wood preservative comprising any suitable biocide and the said hydroperoxide, preferably together with a suitable non-aqueous liquid carrier or solvent. In particular, the composition may be formulated together with a liquid hydrocarbon carrier or solvent, and preferably by using an alkyl or aryl hydrocarbon such as white spirit. Preferably, in such a composition the hydroperoxide and biocide are in a ratio of at least about 10:1 by weight and the biocide preferably is used at a solvent dilution of about 1% by weight.

Since in a wood preservative composition there may be no requirement to choose an organic biocide, in the wood preservative modification the biocide may be selected from any one or more of the organic biocides listed above, namely:

(1) Phenols,
(2) Aldehydes,
(3) Heterocyclic compounds,
(4) Quaternary ammonium compounds, and/or
(5) Cationic compounds.

More preferably, however, the biocide is one of the anti-fungal compounds known for use in wood preservation, whether used alone or in conjunction with one or more other organic biocides as described above. In particular, the biocide for the wood preservative composition may be a heavy metal biocide, for example, tri-butyl tin oxide or the like. Additionally or alternatively, where lower toxicity is of importance the biocide may be o-phenylphenol or the like.

Furthermore, compositions based on cumene hydroperoxide are preferred since they are less liable to be prone to leaching. Also, the organic and/or other biocide should be such as provides biocidal activity since usually biostasis is not sufficient in the context of wood preservation. On the other hand, all of these criteria having been met, the wood preservative composition otherwise may be formulated in accordance with the preceding description.

As a specific Example of a wood preservative composition, the following may be given:

Example 27

A composition was formulated by blending as follows:

| Ingredients | Percent by weight |
|---|---|
| Cumene hydroperoxide | 1 |
| Tri-butyl tin oxide | 0.1 |
| Hydrocarbon solvent (white spirit) | Balance to 100% |

The above composition is useful as a wood preservative and may be applied to wood to be preserved by brush or the like. A similar composition may be prepared using o-phenylphenol in place of the tri-butyl tin oxide.

In our co-pending British Patent Application No. 83-34422 there is described and claimed a composition for use in preserving hydrocarbon products such as aviation and marine fuel oils, which composition comprises an organic biocide containing carbon, hydrogen and oxygen only or a mixture of two or more such biocides, and at least one hydroperoxide of the formula:

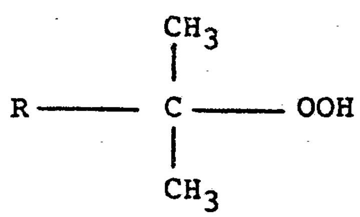

I

wherein R is an aliphatic or aromatic hydrocarbon group, optionally together with a carrier solvent which is compatible with the product to be preserved. In the case of a marine fuel oil preservative the composition may also contain a small amount (usually less than 1% by weight) of a biocide containing nitrogen and/or sulphur.

## CLAIMS

1. A composition for use as a preservative or disinfectant, which composition comprises one or more organic biocides selected from phenols, aldehydes, heterocyclic compounds, quaternary ammonium compounds and cationic compounds, and at least one hydroperoxide of the formula:

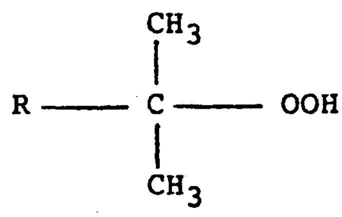

I

wherein R is an aliphatic or aromatic hydrocarbon group.

2. A composition according to claim 1, wherein the organic biocide is an aldehyde, a dioxane, an isothiazolinone, an oxazolidine and/or a phenol, including phenol itself, naphthols and indanols, such as a phenol of the formula:

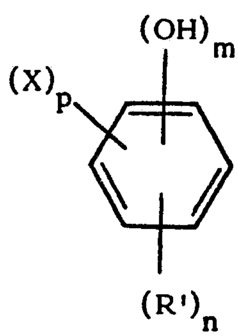

II

3. A composition according to claim 2, wherein the phenol is one or more phenols of formula II wherein p is 0, R' is alkyl or aryl and m is 1, for example, one or more alkyl phenols such as a heptyl phenol, a propyl phenol, o-, m- or p-cresol or a xylenol, or o-phenylphenol, or the phenol is one of formula II wherein R' is hydrogen or alkyl, m and n are 1 and X is bromo, chloro or iodo, such as 4-chlorophenol, 2,4-dichlorophenol, 2,4,6-trichlorophenol, or 4-chloro-o-cresol, or the phenol is o-benzyl-p-chlorophenol.

4. A composition according to any one of the preceding claims, wherein the organic biocide is formaldehyde, a formaldehyde release agent, glyoxal, 6-acetoxy-2,4-dimethyl-m-dioxane, a halo- and/or alkyl-substituted isothiazolinone, N,N'-methylene-bis(5-methyl-oxazolidine), a chlorhexidine, or one or more alkyl p-hydroxybenzoates.

5. A composition according to any one of the preceding claims, wherein the hydroperoxide of formula I is tertiary butyl hydroperoxide (R=methyl), tertiary amyl hydroperoxide (R=ethyl) or cumene hydroperoxide (R=phenyl).

6. A composition according to any one of the preceding claims, which includes at least about 0.1% by weight of an organic biocide, and up to about 5% by weight of hydroperoxide of formula I, preferably no more than about 1% by weight of hydroperoxide and, where the composition is a disinfectant, up to about 40% by weight of organic biocide and, where the composition is a preservative, up to about 15% by weight of organic biocide.

7. A composition according to any one of the preceding claims, wherein the weight ratio of organic biocide to hydroperoxide of formula I is from about 100:1 to about 1:25, preferably from about 15:1 to about 1:15, the organic biocide or the hydroperoxide of formula I preferably being in a substantial excess by weight over the other, more preferably in at least about a two-fold excess over the other.

8. A composition according to any one of the preceding claims, which includes water and which is preferably in the form of an aqueous emulsion or dispersion, preferably a composition including water and a surface active agent present in an amount of at least about 10% by weight of the composition, more preferably in an amount of from about 20 to about 30% by weight.

9. A composition according to claim 8, wherein the surface active agent is an alkali metal salt of an alkyl sulphate, such as a sodium salt of a secondary alkyl sulphate or a primary alkyl sulphate, or a sulphonated castor oil, or a mixture thereof.

10. A composition according to any one of the preceding claims for use as a disinfectant, wherein the composition also includes a stabilizer, such as urea,

and/or an alcohol, such as isopropyl alcohol.

11. A modification of a composition according to any one of claims 1 to 9 for use in preserving wood, which composition comprises at least one hydroperoxide of the formula I defined in claim 1, at least one biocide and optionally a non-aqueous liquid carrier or solvent, such as a liquid hydrocarbon.

12. A modified composition according to claim 11, wherein at least one biocide is chosen so as to provide a biocidal rather than a biostatic effect, for example, at least one biocide is o-phenylphenol and/or a heavy metal biocide such as tri-butyl tin oxide, and the hydroperoxide is preferably in an excess over biocide of at least about 10:1.